# EUROPEAN PATENT SPECIFICATION

(11) **EP 2 244 568 B1**
(45) Date of publication and mention of the grant of the patent: **01.04.2015**
(21) Application number: 08868789.2
(22) Date of filing: 06.12.2008
(51) Int. Cl.: D06F 37/10, A01N 25/34, A47L 15/42, A61L 2/232, D06F 37/26, D06F 37/18

(54) **A WASHER**
WASCHVORRICHTUNG
APPAREIL DE LAVAGE

(30) Priority: 31.12.2007 TR 200709236
(43) Date of publication of application: 03.11.2010
(73) Proprietor: Arçelik Anonim Sirketi, 34950 Istanbul (TR)
(72) Inventor: KARAKAYA, Nursel, 34950 Istanbul (TR); AKAR, Bahar, 34950 Istanbul (TR)
(86) International application number: PCT/EP2008/066964
(87) International publication number: WO 2009/083398

(56) References cited:
- EP-A- 1 612 317
- WO-A-2008/097426
- "RÖMPP CHEMIE LEXIKON" 1992, GEORG THIEME VERLAG , STUTTGART, NEW YORK , XP002525002 PAGES 4572-4573, ITEM: THERMOPLASTISCHE ELASTOMERE
- "RÖMPP CHEMIE LEXIKON" 1992, GEORG THIEME VERLAG , STUTTGART, NEW YORK , XP002525003 PAGE 3701, ITEM: PYRITHION
- DATABASE WPI Week 200326 Thomson Scientific, London, GB; AN 2003-264291 XP002525004 & KR 2002 086 436 A (DONG A HWA SUNG CO LTD) 18 November 2002 (2002-11-18)

## Description

The present invention relates to a washer wherein the hygienic conditions in the washing chamber are enhanced.

The washer of the present invention is a dishwasher or a washing machine. In the said washers, a sealing element, for example a gasket or bellows, is used between the door and the washing chamber to provide leak-proofing. The sealing element is generally produced of elastomeric materials such as EPDM rubber. In time, formation of bacterial plaque and mold is observed on the sealing element as a result of repetitious washing processes. The said bacterial plaque or mold creates a problem in terms of the washing process hygiene. The formation of bacterial plaque and mold increases particularly on the folds of the sealing element or in points hard to access by the wash water.

The aim of the present invention is the realization of a washer wherein the formation of bacterial plaque and mold is prevented on the sealing element between the door and the washing chamber.

The washer realized in order to attain the aim of the present invention is explicated in the claims.

In the washer of the present invention, the sealing element, disposed between the door and the washing chamber, contains an antimicrobial additive. The sealing element is produced of TPE and zinc pyrithione compound material. The TPE material is polystyrene / elastomer block copolymer (e.g. SEBS or SBS) or thermoplastic vulcanizate. TPE is a thermoplastic vulcanizate preferably a compound of EPDM and PP.

The sealing element is made by mixing in 95-97% TPE material by weight and 3-5% master batch material by weight. Master batch is a polyolefin carrying material that contains 1-10% zinc pyrithione.

The washer of the present invention can be a dishwasher or a washing machine.

The formation of bacterial plaque and mold on and in the vicinity of the sealing element is prevented by means of the antimicrobial additive substance added into the material of which the sealing element is produced.

The washer realized in order to attain the aim of the present invention is illustrated in the attached figures, where:
Figure 1 - is the perspective view of a washer in the door closed position.
Figure 2 - is the perspective view of a washer in the door open position.
Figure 3 - is the detailed perspective view of a washer door and the sealing element thereon.
Figure 4 - is the view of detail A in Figure 3.

The elements illustrated in the figures are numbered as follows:
1. Washer
2. Body
3. Washing chamber
4. Loading port
5. Door
6. Sealing element

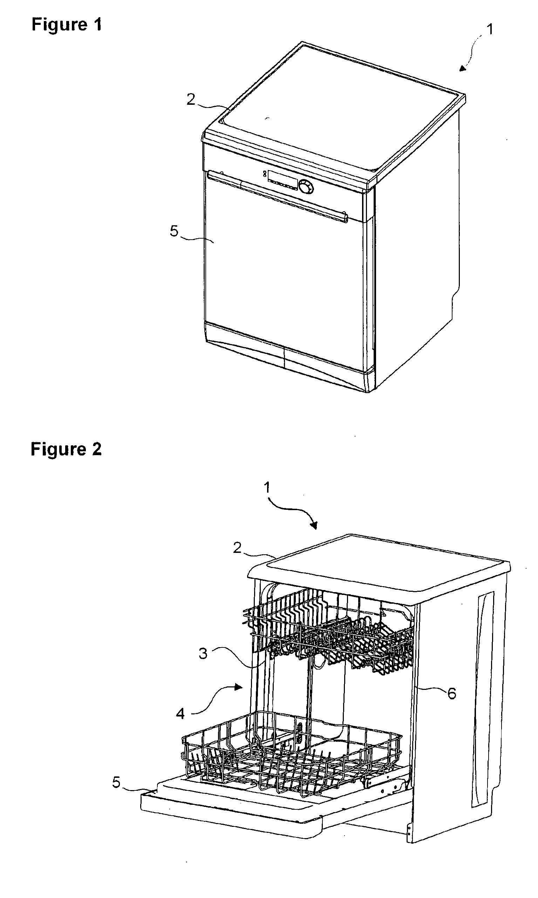

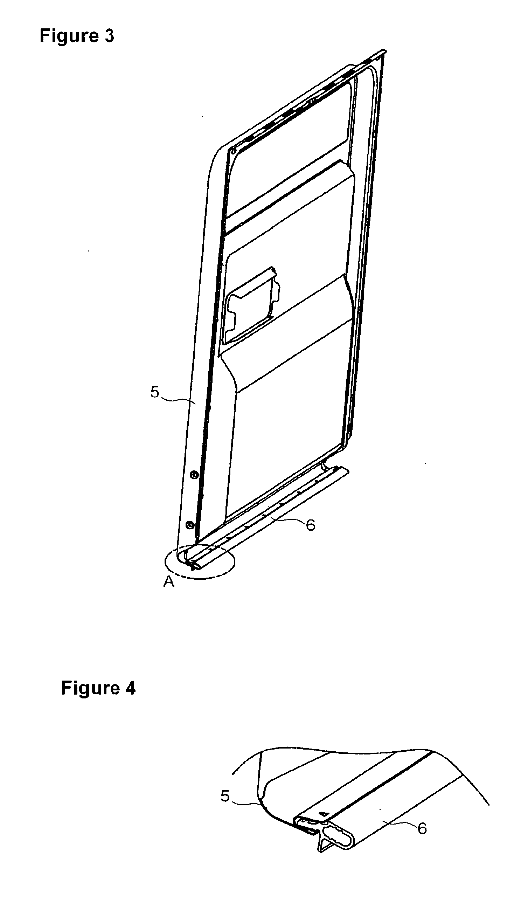

The washer (1) comprises a body (2), a washing chamber (3) wherein the washing process is performed, a loading port (4) for loading the items to be washed into the washing chamber (3), a door (5) that covers the loading port (4) and a sealing element (6) disposed between the loading port (4) and the door (5) (Figure 1, Figure 2, Figure 3 and Figure 4).

The formation of bacterial plaque and mold on the sealing element (6) is prevented by adding an antimicrobial additive substance into the material used for producing the sealing element (6). The sealing element (6) is produced of thermoplastic elastomer (TPE) material. Zinc pyrithione (ZnPT, C₁₀H₈N₂O₂S₂Zn) is used as the antimicrobial substance.

The sealing element (6) is subjected to high temperatures (40°C - 90°C) during the washing process and is in continuous contact with the wash water containing cleaning agents dissolved therein in an environment with a high level of humidity. The temperature, humidity, the cleaning agents and the mechanical effect of the water increases the migration of the zinc in the sealing element (6) to air and the wash water. The rate of zinc pyrithione in the sealing element (6) is determined by taking into consideration the environmental conditions in the sealing element (6) region during the washing process so that the sealing element (6) can sustain the desired antimicrobial effect during the useful life of the washer (1). Another factor considered in determining the rate of zinc pyrithione is the amount of zinc mixed into the wash water. Some portion of the zinc mixed into the wash water remains on the washed item and the remainder is discharged together with the wash water. Accordingly, the amount of zinc has to be at a level not hazardous to human health and the environment.

TPE provides advantages in recyclability and ease of production. TPE material is polystyrene/elastomer block copolymer (e.g. SEBS or SBS) or thermoplastic vulcanizate (EPDM/PP). TPE material used in the sealing element (6) is a thermoplastic vulcanizate (TPV) preferably obtained from a compound of EPDM and polypropylene (PP) and is in granule form.

In the sealing element (6) a master batch material carrying polyolefin, containing 1-10% zinc pyrithione is used as the antimicrobial additive substance.

The sealing element (6) is produced by mixing TPE material and the antimicrobial additive substance in a master batch form, melting by heating and forming by extrusion or injection methods.

For determining the amount of antimicrobial additive substance to be included in the sealing element (6), samples are produced by using 3% and 5% antimicrobial material respectively and the samples are subjected to various tests for assessing the rate of zinc pyrithione that will provide the continuity of antimicrobial effect in the sealing element (6) and the migration of zinc at a safe level health-wise.

### Tests

The method of the present invention is applied on various samples and these samples are subjected to some tests in order to measure the antimicrobial effects and endurances. The bacterial plaque adherence test (JIS Z 2801: 2000E Film Adherence Method - AATC Test Method 100) is a frequently used method since it determines the effect on bacteria cells quantitatively in an environment not containing nutrients. During the test, a solution containing 10⁵- 10⁶ cfu bacteria is engrafted on samples of 50mm x 50mm. A waiting period of 24 hours is applied at a temperature of 23-35° C depending on the type of bacteria used (Coliform bacillus - E.c., staphylococcus - S.a., etc.). The number of bacteria still alive (cfu= colony forming unit) is counted by plate counting method. The logarithmic decrease in the number of bacteria as compared with the reference plate is described as the antibacterial effectiveness percentage. Log 2 decrease corresponds to 99% sufficient effectiveness, log 3 decrease to 99.9% and log 4 decrease to 99.99%.

The antimicrobial material field of influence test (Agar Diffusion Methods (Kirby- Bauer) - AATC Test Method 147), is also called Zone of Inhibition method. It is a qualitative evaluation method. The samples are placed on the agar solution (3 mm), the nutrient medium, together with the target microorganisms and incubated at 20-30°C for 24 hours. The clean halo area formed around the sample shows antimicrobial effectiveness. The test does not yield results if the antimicrobial material migration is too slow. The applicability of the test depends on the diffusion rate of the antimicrobial material made of polymer, solubility and the rate of diffusion in agar.

In operation tests, the washer (1) is loaded with items to be washed having standard dirtiness levels and the standard washing program of the washer (1) is activated. 35°C and 70°C are used as the washing temperatures. This process is repeated for 1000 cycles. The antimicrobial effectiveness of the sealing element (6) is measured by the JIS Z 2801: 2000E method before the first cycle and after the 250^{th}, 600^{th} and 900^{th} cycles. For each condition, tests are carried in three washers (1) and the average of the results is given in the following tables:

The samples and test results are given below:

### Sample 1:

In this sample, the sealing element (6) includes antimicrobial additive substance in master batch form 3% by weight, TPE material 97% by weight. Test results are given in Table A.

Table A:

**Table 1**

| Washing Temperature | No. of Cycles | Test | Result | Comment |
|---|---|---|---|---|
| - | 0 | JIS Z 2801: 2000E | Log 3.84 | Effectiveness sufficient |
| - | 0 | Agar Diffusion Methods | 16.4 cm² | Effectiveness present |
| 35 | 250 | JIS Z 2801: 2000E | Log 2.60 | Effectiveness sufficient |
| 35 | 600 | JIS Z 2801: 2000E | Log 2.70 | Effectiveness sufficient |
| 35 | 900 | JIS Z 2801: 2000E | Log 3.66 | Effectiveness sufficient |
| 70 | 250 | JIS Z 2801: 2000E | Log 2.35 | Effectiveness sufficient |
| 70 | 600 | JIS Z 2801: 2000E | Log 3.72 | Effectiveness sufficient |
| 70 | 900 | JIS Z 2801: 2000E | Log 3.50 | Effectiveness sufficient |

The tests carried out show that this sample is successful in preventing the reproduction of bacteria and molds.

### Sample 2:

In this sample, the sealing element (6) includes antimicrobial additive substance in master batch form 5% by weight, TPE material 95% by weight. Test results are given in Table B.

Table B:

**Table 2**

| Washing Temperature | No. of Cycles | Test | Result | Comment |
|---|---|---|---|---|
| - | 0 | JIS Z 2801: 2000E | Log 3.75 | Effectiveness sufficient |
| - | 0 | Agar Diffusion Methods | 19.7 cm² | Effectiveness present |
| 70 | 250 | JIS Z 2801: 2000E | Log 4.18 | Effectiveness sufficient |
| 70 | 600 | JIS Z 2801: 2000E | Log 3.67 | Effectiveness sufficient |

The tests carried out show that this sample is successful in preventing the reproduction of bacteria and molds. The sealing element (6) contains 95-97% TPE material by weight and antimicrobial additive substance in master batch form 3-5% by weight. The sealing element (6) contains zinc pyrithione of 0.03 - 0.50% by weight since the antimicrobial material in master batch form contains zinc pyrithione of 1-10% by weight.

In the washer (1) of the present invention, during the washing process, the antimicrobial substance in the sealing element (6) migrates towards the surface of the sealing element (6) and neutralizes the bacteria and the molds on the surface of the sealing element (6). The antimicrobial substance depolarizes the electro-potential of the cell membrane and prevents bacterial / fungal conveyance on the surface of the sealing element (6). Consequently, the formation of bacterial plaque and mold on the surface of the sealing element (6) is prevented.

The zinc pyrithione (ZnPT) - TPE mixture provides considerable advantages over the widely used mixture of EPDM - silver mixture. EPDM used in the sealing elements (6) is a thermoset material which is produced by vulcanizing with sulfur. The desired antimicrobial effect on the sealing element (6) cannot be maintained by the use of silver based materials since sulfur interacts with silver based antimicrobial additive substance. Therefore, in order to use silver based antimicrobial materials as additives, vulcanization has to be done with peroxide. However, vulcanization with peroxide results in the problem of bad odor. Vulcanization with peroxide is avoided since this odor is not desired to emanate in the dishwasher or the washing machine by the consumers.

In an embodiment of the present invention, the washer (1) is a dishwasher and the sealing element (6) is the gasket between the loading port (4) and the door (5).

In another embodiment of the present invention, the washer (1) is a washing machine and the sealing element (6) is the bellows between the loading port (4) and the door (5).

A hygienically enhanced washing medium is provided by the sealing element (6) of the present invention containing an antimicrobial material additive.

## Claims

1. A washer (1) that comprises a body (2), a washing chamber (3) wherein the washing process is performed, a loading port (4) for loading the items to be washed into the washing chamber (3), a door (5) that covers the loading port (4) and a sealing element (6) disposed between the loading port (4) and the door (5) **and characterized in that** the sealing element (6) is produced of thermoplastic elastomer (TPE) material and zinc pyrithione (ZnPT) as antimicrobial material.

2. A washer (1) as in Claim 1, **characterized in that** the thermoplastic elastomer (TPE) material is polystyrene/elastomer block copolymer.

3. A washer (1) as in Claim 1, **characterized in that** the thermoplastic elastomer (TPE) material is a thermoplastic vulcanizate (TPV) derived from EPDM and polypropylene (PP).

4. A washer (1) as in any one of the Claims 1 to 3, **characterized in that** the sealing element (6) is produced by using 95-97% TPE material by weight and 3-5% by weight antimicrobial additive substance in master batch form.

5. A washer (1) as in Claim 4, **characterized in that** the sealing element (6) contains 1-10% zinc pyrithione by weight and polyolefin as the carrier.

6. A washer (1) according to any of claims 1-4, **characterized by** the sealing element (6) that contains 0.03% - 0.50% zinc pyrithione by weight.

7. A washer (1) as in any one of the above Claims, which is a dishwasher.

8. A washer (1) as in any one of the Claims 1 to 6, which is a washing machine.

## Patentansprüche

1. Wasch-/Spülvorrichtung (1), umfassend einen Gehäusekörper (2), eine Wasch-/Spülkammer (3), in der der Wasch-/Spülvorgang durchgeführt wird, eine Ladeöffnung (4) zum Laden von zu waschenden/spülenden Artikeln in die Wasch-/Spülkammer (3), eine Tür (5), die die Ladeöffnung (4) abdeckt, und ein Dichtungselement (6), das zwischen der Ladeöffnung (4) und der Tür (5) angeordnet ist, und **dadurch gekennzeichnet, dass** das Dichtungselement (6) aus thermoplastischem Elastomer(TPE)-Material und Zinkpyrithion (ZnPT) als antimikrobiellem Material hergestellt ist.

2. Wasch-/Spülvorrichtung (1) nach Anspruch 1, **dadurch gekennzeichnet, dass** das thermoplastische Elastomer(TPE)-Material Polystyrol/Elastomer-Blockcopolymer ist.

3. Wasch-/Spülvorrichtung (1) nach Anspruch 1, **dadurch gekennzeichnet, dass** das thermoplastische Elastomer(TPE)-Material ein thermoplastisches Vulkanisat (TPV) ist, das von EPDM und Polypropylen (PP) gewonnen wird.

4. Wasch-/Spülvorrichtung (1) nach einem der Ansprüche 1 bis 3, **dadurch gekennzeichnet, dass** das Dichtungselement (6) mit 95-97 Gew.-% TPE-Material und 3-5 Gew.-% antimikrobieller Zusatzsubstanz in der Vormischungsform hergestellt wird.

5. Wasch-/Spülvorrichtung (1) nach Anspruch 4, **dadurch gekennzeichnet, dass** das Dichtungselement (6) 1-10 Gew.-% Zinkpyrithion und Polyolefin als Träger enthält.

6. Wasch-/Spülvorrichtung (1) nach einem der Ansprüche 1-4, **dadurch gekennzeichnet, dass** das Dichtungselement (6) 0,03 bis 0,50 Gew.-% Zinkpyrithion enthält.

7. Wasch-/Spülvorrichtung (1) nach einem der vorangehenden Ansprüche, die eine Spülmaschine ist.

8. Wasch-/Spülvorrichtung (1) nach einem der Ansprüche 1 bis 6, das eine Waschmaschine ist.

## Revendications

1. Un laveur (1) qui comprend un corps (2), une chambre de lavage (3) où le processus de lavage est effectué, un orifice de chargement (4) pour le chargement des articles à laver dans la chambre de lavage (3), une porte (5) qui couvre l'orifice de chargement (4) et un élément d'étanchéité (6) disposé entre l'orifice de chargement (4) et la porte (5) **et caractérisée en ce que** l'élément d'étanchéité (6) est produit en élastomère thermoplastique (TPE) et en pyrithione de zinc (ZnPT) comme matériau antimicrobien.

2. Un laveur (1) selon la Revendication 1, **caractérisée en ce que** l'élastomère thermoplastique (TPE) est un copolymère en bloc de polystyrène/élastomère.

3. Un laveur (1) selon la Revendication 1, **caractérisée en ce que** l'élastomère thermoplastique (TPE) est un vulcanisat thermoplastique (TPV) dérivé d'EPDM et polypropylène (PP).

4. Un laveur (1) selon l'une quelconque des revendications de 1 à 3, **caractérisée en ce que** l'élément d'étanchéité (6) est produit en utilisant 95-97% de matière TPE en poids et 3-5% en poids de substance additive antimicrobien en forme de mélange-maître.

5. Un laveur (1) selon la Revendication 4, **caractérisé en ce que** l'élément d'étanchéité (6) contient de 1-10% de pyrithione de zinc en poids et polyoléfine comme porteur.

6. Un laveur (1) selon l'une quelconque des revendications de 1 à 4, **caractérisé par** l'élément d'étanchéité (6) qui 0.03%-0.50% de pyrithione de zinc en poids.

7. Un laveur (1) selon l'une quelconque des revendications précédentes, qui est un lavevaisselle.

8. Un laveur (1) selon l'une quelconque des revendications de 1 à 6, qui est un lave-linge.
